# FASCICULE DE BREVET EUROPEEN

(11) **EP 0 764 444 B1**
(45) Date de publication et mention de la délivrance du brevet: **13.03.2002**
(21) Numéro de dépôt: 96401784.2
(22) Date de dépôt: 13.08.1996
(51) Int. Cl.: A61K 38/06, A61K 7/06, A61K 7/48, C07K 5/09, C07K 105/00, A61P 29/00, A61P 17/14

(54) **Utilisation d'au moins un peptide contenant le tripeptide Lys-DPro-Arg pour la préparation d'un médicament pour le traitement de l'inflammation**
Verwendung mindestens eines die Tripeptidsequenz Lys-DPro-Arg enthaltenden Peptids zur Herstellung eines Arzneimittels zur Behandlung einer Entzündung
Use of a least one peptide containing the Lys-DPro-Arg tripeptide for the preparation of a medicament for treating inflammation

(30) Priorité: 19.09.1995 FR 9510977
(43) Date de publication de la demande: 26.03.1997
(73) Titulaire: L'OREAL, 75008 Paris (FR)
(72) Inventeur: Mahe, Yann, 91390 Morsang sur Orge (FR); Buan, Bruno, 93170 Bagnolet (FR)
(74) Mandataire: Andral, Christophe André Louis

(56) Documents cités:
- EP-A- 0 335 662
- WO-A-88/00833
- EUR. J. PHARMACOL. (1994), 258(1-2), 131-8, 1994, XP000570480 OLUYOMI, ADEMOLA O. ET AL: "Antinociceptive activity of peptides related to interleukin -1.beta.-(193-195), Lys-Pro-Thr"
- PEPTIDES (FAYETTEVILLE, N. Y.) (1991), 12(4), 767-71, 1991, XP000570459 HILTZ, M. E. ET AL: "Anti-inflammatory activity of.alpha.-MSH(11-13) analogs: influences of alteration in stereochemistry"

## Description

La présente invention concerne l'utilisation à titre de principe actif, dans un milieu physiologiquement acceptable, pour la préparation d'un médicament, d'une quantité efficace d'au moins un peptide contenant le tripeptide Lysine-Proline-Valine, ou de tout équivalent biologique fonctionnel, dans lequel le résidu Proline est sous la forme de son isomère optique dextrogyre, destinée à traiter l'inflammation.

L'inflammation est un ensemble de réactions biologiques qu'on retrouve dans toute l'échelle animale. Chez l'homme, deux malades sur trois présentent un syndrome inflammatoire. L'inflammation peut être localisée.
Elle peut se définir comme la première réponse à toute agression locale par une série de réactions non spécifiques déclenchées quelle que soit la cause initiale et se déroulant en trois temps : vasculaire, cellulo-vasculaire et fibrose tissulaire. Gonflement, douleur, rougeur, chaleur sont les termes que l'on peut utiliser pour décrire l'inflammation localisée. Ceux-ci sont généralement dus à l'infiltration des tissus blessés par un oedème et/ou à la vasodilatation des capillaires.

Les signes de l'inflammation peuvent aller jusqu'à la fièvre, un état de malaise général et/ou une augmentation de la concentration de certaines protéines de plasma sanguin.
C'est un phénomène qui implique entre autres une série de réactions cellulaires locales et la libération de cytokines et autres médiateurs tels que la substance P, les prostaglandines, l'histamine ou encore la sérotonine.
Elle se manifeste par une modification du flot sanguin avec, au niveau du site agressé, une augmentation de la perméabilité vasculaire entraînant une fuite de protéines plasmatiques et de cellules vers le fluide extracellulaire, ainsi qu'une extravasation de leucocytes, principalement des leucocytes neutrophiles et de macrophages vers le site inflammatoire.

Ces phénomènes sont en fait le résultat de l'action des médiateurs de l'inflammation.

Parmi les facteurs impliqués dans ces phénomènes inflammatoires, on peut citer les cytokines dont en particulier l'interleukine 1-α, l'interleukine 1-β, l'interleukine 6, les facteurs de nécrose tumorale α et β (TNF-α et -β), les chémokines comme l'interleukine 8 ou le facteur chimiotactique et activateur des monocytes (MCAF), ou encore d'autres facteurs chimiotactiques responsables du recrutement des cellules lymphocytaires, monocytaires, de Langerhans ou basophiles au niveau du site inflammatoire, tels que les leukotriènes B-4, ou encore d'autres facteurs impliqués dans la cascade inflammatoire, tels que l'acide arachidonique, ou les prostaglandines, dont en particulier les prostaglandines E2.

Les phénomènes inflammatoires sont associés à de nombreuses pathologies.
On peut citer à titre d'exemple l'érythème solaire, le prurit, l'érythème noueux, l'urticaire, la mastocytose systémique, le psoriasis, les piqûres d'insectes, d'autres affections dermatologiques comme la polychondrite atrophiante, l'érythermalgie, la nécrobiose lipoïdique. On peut encore citer le lupus érithèmateux disséminé, les spondylarthropathies ou les atteintes articulaires des entéropathies chroniques.

On recherche depuis de nombreuses années, dans l'industrie pharmaceutique, des substances permettant de traiter l'inflammation.

A cet égard, il a été récemment proposé d'utiliser une quantité suffisante d'un dérivé de l'hormone stimulatrice des mélanocytes de type α (α-MSH) ou Mélanotropine et particulièrement le peptide contenant le tripeptide Lysine-Proline-Valine (US 5028592, US 5157023).
Il a cependant été montré que la forme optique des isomères utilisés dans la composition du tripeptide avait une grande importance. Ainsi, il a été montré que lorsque le résidu Proline apparaît dans le tripeptide sous sa forme d'isomère optique dextrogyre (Dpro), le tripeptide ou le peptide contenant le tripeptide perdait toute efficacité dans le traitement de l'inflammation (Hiltz et al. Peptides, vol. 12, pp 767-771, 1991).

Or, la demanderesse vient maintenant de découvrir, après d'importantes recherches menées sur la question, qu'un peptide contenant le tripeptide Lysine-Proline-Valine, dans lequel le résidu Proline apparaît dans le tripeptide sous sa forme d'isomère optique dextrogyre (DPro), ou tout équivalent biologique fonctionnel est actif dans le traitement de l'inflammation.
Par équivalent biologique fonctionnel, on entend un peptide fonctionnellement équivalent en terme de fonction biologique dont l'un au moins des résidus d'acide aminé peut avoir été changé pour un résidu d'acide aminé ayant un index hydropathique similaire.

Cette découverte est à la base de la présente invention.

Ainsi, l'invention concerne l'utilisation pour la préparation d'un médicament destinés à traiter l'inflammation d'au moins un peptide contenant le tripeptide Lysine-Proline-Valine, dans lequel le résidu Proline apparaît sous sa forme d'isomère optique dextrogyre (DPro), ou de tout équivalent biologique fonctionnel.

Dans le domaine des acides aminés, la géométrie des molécules est telle qu'elles peuvent théoriquement se présenter sous la forme d'isomères optiques différents. Il existe en effet une conformation moléculaire de l'acide aminé (aa) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa).

La nature n'a retenu pour les acides aminés naturels que la conformation lévogyre. En conséquence un peptide d'origine naturelle ne sera constitué que d'acides aminés de type L-aa.
Cependant, la synthèse chimique en laboratoire permet de préparer des d'acides aminés ayant les deux conformations possibles. A partir de ce matériel de base il est possible d'incorporer lors de la synthèse de peptides aussi bien des d'acides aminés sous la forme d'isomères optiques dextrogyre ou lévogyre.
On peut ainsi incorporer lors de la synthèse de peptides outre le résidu D-Proline (D-Pro), des résidus d'acides aminés Lysine ou Valine indifféremment sous leur forme D-Lysine (D-Lys), L-Lysine (L-Lys), D-Valine (D-Val) ou L-Valine (L-Val).

Ainsi, l'invention concerne plus particulièrement l'utilisation d'une quantité suffisante du peptide tel que défini précédemment, caractérisée par le fait que les résidus Lysine ou Valine du tripeptide Lysine-(D)Proline-Valine constituant le peptide sont indifféremment sous la forme d'isomères optiques dextrogyre ou lévogyre.

On peut citer ainsi les peptides contenant au moins l'un des tripeptides suivant :
D-Lys-D-Pro-D-Val,
D-Lys-D-Pro- L-Val,
L-Lys-D-Pro-D-Val,
L-Lys-D-Pro-L-Val.

Le tripeptide se trouve avantageusement à l'extrémité C-terminale du peptide.

Préférentiellement, le peptide utilisé selon l'invention est le tripeptide Lysine-Proline-Valine dans lequel le résidu Proline apparaît sous sa forme d'isomère optique dextrogyre (DPro).

De préférence également, le peptide utilisé selon l'invention contient le tripeptide Lysine-Proline-Valine dans lequel les résidus Lysine, Proline et Valine apparaissent sous la forme d'isomères optiques dextrogyres (DLys-DPro-DVal).

Selon l'invention, il peut bien entendu être utilisé plus d'un peptide. Dans ce cas, le mélange de peptides peut être constitué par l'une des combinaisons possibles des peptides ci-dessus décrits.

Dans le texte qui va suivre, de manière générale, le terme Proline s'entend comme désignant le résidu Proline sous sa forme d'isomère optique dextrogyre (DPro) et le terme "peptide" couvre aussi bien le "peptide contenant le tripeptide Lysine-Proline-Valine, ou tout équivalent biologique fonctionnel", que le "tripeptide Lysine-Proline-Valine" isolé, dans lesquels le résidu Proline est sous sa forme d'isomère optique dextrogyre (DPro).

Il se peut que pour des questions de résistance à la dégradation il soit nécessaire d'utiliser selon l'invention une forme protégée du peptide. La forme de la protection doit évidemment être une forme biologiquement compatible. De nombreuses formes de protections biologiquement compatibles peuvent être envisagées comme par exemple l'acylation ou l'acétylation de l'extrémité amino-terminale ou l'amidation de l'extrémité carboxy-terminale.

Ainsi, l'invention concerne une utilisation telle que précédemment définie, caractérisée par le fait que le peptide est sous une forme protégée ou non.

De préférence, on utilise selon l'invention une protection basée soit sur l'acylation ou l'acétylation de l'extrémité amino-terminale, soit sur l'amidation de l'extrémité carboxy-terminale soit encore sur les deux.

La quantité efficace d'actif correspond à la quantité nécessaire pour obtenir le résultat désiré.

Plus particulièrement, le peptide est présent dans une quantité telle que le tripeptide Lysine-Proline-Valine peut être utilisé à une concentration comprise entre 10⁻¹² M et 1 M et de préférence comprise entre 10⁻⁶ M. et 10⁻¹ M.

Il est clair que l'homme du métier sait ajuster cette quantité de matériel selon qu'il utilise le peptide contenant le tripeptide Lysine-Proline-Valine, ou tout équivalent biologique fonctionnel ou le tripeptide Lysine-Proline-Valine.

La composition selon l'invention peut être administrée par voie parentérale, entérale ou encore par voie topique. De préférence, la composition peut être administrée par voie topique.

Le milieu physiologiquement acceptable dans lequel le peptide est utilisé selon l'invention peut être anhydre ou aqueux. On entend par milieu anhydre, un milieu solvant contenant moins de 1% d'eau. Ce milieu peut être constitué d'un solvant ou d'un mélange de solvants choisi plus particulièrement parmi les alcools inférieurs en C₂-C₄ comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 4 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

Il est possible que le milieu physiologiquement acceptable puisse contenir d'autres adjuvants habituellement utilisés dans le domaine pharmaceutique, tels que des agents tensioactifs, des agents épaississants ou gélifiants, des agents conservateurs, des agents alcalinisants ou acidifiants bien connus dans l'état de la technique, et en quantités suffisantes pour obtenir la forme de présentation désirée, notamment de lotion plus ou moins épaissie, de gel, d'émulsion, ou de crème. L'utilisation peut éventuellement se faire sous une forme pressurisée en aérosol ou vaporisée à partir d'un flacon pompe.

Il est possible aussi d'utiliser en association avec le peptide, des composés déjà décrits pour leur activité anti-inflammatoire.

On peut plus particulièrement citer les glucocorticoïdes, la vitamine D et ses dérivés et les anti-inflammatoires non stéroïdiens.

L'utilisation du peptide selon l'invention peut se faire par application topique d'une composition contenant une quantité efficace d'au moins un peptide contenant le tripeptide Lysine-Proline-Valine dans lequel le résidu Proline apparaît sous sa forme d'isomère optique dextrogyre (DPro), sur une partie du corps présentant les symptômes de l'inflammation.

Ainsi, la présente invention a également pour objet l'utilisation, caractérisé en ce que l'on applique sur la peau, sur le cuir chevelu et/ou sur les muqueuses des zones cutanées présentant les symptômes de l'inflammation, d'une composition cosmétique contenant une quantité efficace d'au moins un peptide contenant le tripeptide Lysine-Proline-Valine dans lequel le résidu Proline apparaît sous sa forme d'isomère optique dextrogyre (DPro).

L'utilisation selon l'invention peut être mise en oeuvre notamment en appliquant les compositions telles que définies ci-dessus selon la technique d'utilisation habituelle de ces compositions. Par exemple : application de crèmes, de gels, de sérums, de lotions, de laits de démaquillage ou de compositions anti-solaires sur la peau ou sur le cuir chevelu, de shampooings, ou encore application de dentifrice sur les gencives.

On va maintenant donner à titre d'illustration des exemples qui ne sauraient limiter en aucune façon la portée de l'invention. Dans les compositions les proportions indiquées sont des pourcentages en poids du poids total de la composition.

### Exemple 1: Activité dose-réponse du tripeptide Ac - LPV - NH₂* sur la production d'interleukine 1α dans le surnageant de cheveux épilés provenant d'un alopécique inflammatoire :

Des cheveux épilés sont prélevés dans la zone du vertex sur un alopécique inflammatoire volontaire. Ils sont placés dans un milieu de survie Williams' E (vendu par la société Gibco BRL) contenant de la pénicilline G (100 unités /ml), de la streptomycine-S (100µg/ml), de l'Amphotericine (250 ng/ml), en présence ou non (contrôle), du tripeptide Ac - LPV - NH₂* synthétisé à façon par la société Neosystem S.A. (Strasbourg) aux doses indiquées. Après 20 heures d'incubation, les surnageants de culture sont collectés dans un tube puis centrifugés 5 minutes à 14000 tours/minute (centrifugeuse Eppendorff, modèle 5415C). Les surnageants sont alors collectés dans un tube propre et placés à 4°C.
La concentration en interleukine 1-α est ensuite évaluée sur 100µl de surnageant au moyen d'un kit ELISA Biotrak commercialisé par la société Amersham, suivant les instructions du fournisseur.

### Résultats:

| | Dose | IL-1α | %d'inhibition |
|---|---|---|---|
| Contrôle | | 21.8 pg/ml | |
| Ac-LPV-NH₂* | 10µM | 6.1 pg/ml | 72% |
| Ac-LPV-NH₂* | 1µM | 11.1 pg/ml | 49% |

### Exemple 2 :

### Inhibition de l'expression des ARN messagers de cytokines pro-inflammatoires et inflammatoires en réponse au tripeptide Ac - LPV - NH₂*.

Dix cheveux épilés sont prélevés dans la zone du vertex sur un alopécique inflammatoire volontaire. Ils sont placés dans un milieu de survie Williams' E (vendu par la société Gibco BRL) contenant de la pénicilline G (100 unités /ml), de la streptomycine-S (100µg/ml), de l'Amphotericine (250 ng/ml), en présence (lot traité) ou non (lot contrôle) du tripeptide Ac - LPV - NH₂* synthétisé à façon par la société Neosystem S.A., Strasbourg .
Après 3h30 d'incubation, les ARN messagers correspondant à ces deux lots de cheveux sont purifiés à partir d'un kit « quick prep mRNA purification Kit » commercialisé par la société Pharmacia. Des ADN complémentaires de ces ARNm sont ensuite préparés au moyen d'un kit de reverse transcription commercialisé par la société Pharmacia en suivant les instructions du fournisseur puis soumis à une étape de réaction de polymérisation en chaîne (PCR) en utilisant des amorces spécifiques des ARNm de la Glycéraldéhyde 3-Phosphate Déshydrogénase (GAPDH), de l'IL-1 alpha, du récepteur à l'IL-1 de type 1 et du récepteur à l'IL-1 de type 2. Les quantités d'ADN amplifié sont ensuite évaluées par électrophorèse sur gel d'agarose à 1.5% en présence de bromure d'éthidium. L'intensité des bandes est estimée sous irradiation ultraviolette au moyen d'une caméra vidéo et d'un logiciel d'analyse (Bioprofil TM) commercialisés par la société Vilbert-Lourmat. L'intensité des bandes obtenues avec les amorces IL-1α, IL-1R1, IL-1R2 est divisée par l'intensité des bandes obtenue avec les amorces amplifiant le standard interne GAPDH.

### Résultats:

| % d'expression | IL-1α | IL-1R1 | IL-1R2 |
|---|---|---|---|
| Lot contrôle | 100% | 100% | 100% |
| Lot traité | 0% | 26% | 21% |

### Exemple 3 :

### Effet inhibiteur du Ac - LPV - NH₂* sur l'expression des ARNm de l'IL-1α calcul du ratio IL-1α/GAPDH:

Sur deux donneurs différents, 5 cheveux sont épilés puis incubés 20 heures à 37°C (5% CO2) dans du milieu E de Williams' supplémenté en antibiotiques, en glutamine et en présence d' Ac - LPV - NH₂* aux concentrations indiquées. Un contrôle est réalisé dans lequel il n'y a pas de peptide ajouté. Les résultats sont exprimés en % du contrôle.

| Donneurs | | A | B |
|---|---|---|---|
| Contrôle | | 100% | 100% |
| Ac-LPV-NH₂* | 10µM | 28% | 28.7% |
| Ac-LPV-NH₂* | 1µM | 51% | 62% |
| Ac-LPV-NH₂* | 0.1µM | ND | 53% |
| ND : Non déterminé | | | |

### Exemple 4 :

### Mesure de l'inhibition de la production de PGE2 par des cellules de papille catagène en culture in vitro :

Les cellules (1000 par puits), au passage 24, sont incubées dans du milieu 199 commercialisé par la société GIBCO en présence de 1% de sérum de veau foetal et d'antibiotiques. 20 heures après, le milieu est remplacé par un milieu identique mais contenant les tripeptides à évaluer à la concentration finale de 10µM. 5 heures plus tard, l'interleukine 1α est ajoutée à la concentration finale de 10 ng/ml. 20 heures après, les taux de PGE2 produits par les cellules de papille en culture sont évalués au moyen d'un kit Biotrak commercialisé par la société Amersham en suivant les instructions du fournisseur. Cette méthode permet ainsi d'évaluer l'effet inhibiteur de ces tripeptides sur la production de PGE2 induites par une cytokine pro-inflammatoire: l'interleukine 1α.

| | Dose | PGE2 (pg/ml) | |
|---|---|---|---|
| Contrôle | | 9.2 | |
| IL-1α | | 100.2 | |

| | | | % inhibition |
|---|---|---|---|
| Ac-LPV-NH₂* | 10µM | 22.0 | 78% |
| Ac-L-P-V-NH₂** | 10µM | 10.0 | 90% |
| Ac-L-(D)P-V-NH₂*** | 10µM | 24.6 | 75% |

Ces résultats indiquent, de façon surprenante, une capacité anti-inflammatoire comparable des tripeptides contenant soit la forme (D)-Pro soit la forme naturelle (L)Pro.

### Exemple 5 : Exemples de compositions contenant le tripeptide Ac - LPV - NH₂*. Ces compositions sont obtenues par les techniques de préparations classiques et en particulier par simple mélange des ingrédients.

| Composition 1 : Spray : | |
|---|---|
| Ac-LPV-NH₂* | 5.10⁻⁶ g |
| Minoxidil | 0,5 g |
| Ethanol à 95° | 55,1 g |
| Propylène glycol | 22,8 g |
| Parfum | qs |
| Eau déminéralisée | qsp 100 g |

| Composition 2 : Lotion quotidienne : | |
|---|---|
| Ac-LPV-NH₂* | 12,5.10⁻⁶ g |
| 2,4 diaminopyrimidine-3-oxyde | 0,75 g |
| Ethanol à 95° | 30 g |
| Parfum | qs |
| Colorants | qs |
| Eau déminéralisée | qsp 100 g |

| Composition 3 : Gel liposomé : | |
|---|---|
| Natipide II ① (soit 2 g en phospholipides) | 10 g |
| Ac-LPV-NH₂* | 5.10⁻⁵ g |
| Carbomer | 0,25 g |
| Triéthanolamine | qs pH = 7 |
| Conservateurs - | qs |
| Eau déminéralisée | qsp 100 g |

| | |
|---|---|
| ① Mélange Eau/Alcool/Lécithine de la Société Nattermann | |

| Composition 4 : Gel niosomé : | |
|---|---|
| Chimexane NS① | 1,8 g |
| Stearoylglutamate monosodique | 0,2 g |
| Ac-LPV-NH₂* | 7,5.10⁻⁴ g |
| Carbomer | 0,2 g |
| Triéthanolamine | qs pH = 7 |
| Conservateurs | qs |
| Parfums | qs |
| Eau déminéralisée | qsp 100 g |

| | |
|---|---|
| ① Tensioactif non ionique vendu par la société Chimex. | |

| Composition 5 : Lotion niosomée : | |
|---|---|
| Chimexane NL① | 0,475 g |
| Cholestérol | 0,475 g |
| Stearoylglutamate monosodique | 0,05 g |
| Ac-LPV-NH₂* | 10⁻³ g |
| Conservateurs | qs |
| Colorants | qs |
| Parfum | qs |
| Eau déminéralisée | qsp 100 g |

| | |
|---|---|
| ① Tensioactif non ionique vendu par la société Chimex. | |

| Composition 6 : Crème de soin : émulsion H/E | |
|---|---|
| Alcool cétylstéarylique/Alcool cétylstéarylique oxyéthylénée à 33 moles d'oxyéthylène (80/20) | 5 g |
| Monostéarate de glycérol | 1,5 g |
| Alcool cétylique | 0,75 g |
| Huile de vaseline | 10 g |
| Polydimethylsiloxane | 0,75 g |
| Glycérine | 4 g |
| Conservateurs | qs |
| Ac-LPV-NH₂* | 5.10⁻³ g |
| Eau déminéralisée | qsp 100 g |

| Composition 7 : Solution injectable par voie intradermique | |
|---|---|
| Ac-LPV-NH₂* | 0,7 mg |
| Sérum physiologique (NaCl 9 g/H2O qsp 100 ml) | q.s.p. 1 ml |

| | |
|---|---|
| * : Acétyl - (D)Lys - (D)Pro - (D)Val - NH₂ **: Acétyl - (L)Lys - (L)Pro - (L)Val - NH₂ ***: Acétyl - (L)Lys - (D)Pro - (L)Val - NH₂ | |

## Revendications

1. Utilisation pour la préparation d'un médicament, d'une quantité efficace d'au moins un peptide contenant le tripeptide Lysine-Proline-Valine dans lequel le résidu Proline apparaît sous sa forme d'isomère optique dextrogyre (DPro) ou de tout équivalent biologique fonctionnel, destinée à traiter l'inflammation.

2. Utilisation selon la revendication précédente, **caractérisée par le fait que** le tripeptide se trouve à l'extrémité C-terminale du peptide.

3. Utilisation selon l'une des revendications précédentes, **caractérisée par le fait que** le peptide est le tripeptide Lysine-Proline-Valine dans lequel le résidu Proline apparaît sous sa forme d'isomère optique dextrogyre (DPro).

4. Utilisation selon l'une des revendications précédentes, **caractérisée par le fait que** le peptide est le tripeptide Lysine-Proline-Valine dans lequel les résidus Lysine, Proline et Valine apparaissent sous la forme d'isomères optiques dextrogyres (D-Lys-D-Pro-D-Val).

5. Utilisation selon l'une des revendications précédentes, **caractérisée par le fait que** le peptide est sous une forme protégée ou non.

6. Utilisation selon la revendication précédente, **caractérisée par le fait que** la protection consiste en une protection basée soit sur l'acylation ou l'acétylation de l'extrémité amino-terminale, soit sur l'amidation de l'extrémité carboxy-terminale soit encore sur les deux.

7. Utilisation pour la préparation d'un médicament selon l'une quelconque des revendications 1 à 6, **caractérisée par le fait que** le tripeptide Lysine-Proline-Valine dans lequel le résidu Proline apparaît sous sa forme d'isomère optique dextrogyre (DPro) est utilisé à une concentration comprise entre 10⁻¹²M et 1 M et de préférence comprise entre 10⁻⁶ M et 10⁻¹ M.

## Patentansprüche

1. Verwendung einer wirksamen Menge mindestens eines Peptids, das die Tripeptidsequenz Lysin-Prolin-Valin aufweist, worin der Prolinrest in Form des optisch rechtsdrehenden Isomers (DPro) vorliegt, oder beliebiger in der biologischen Funktion äquivalenter Peptide zur Herstellung eines Arzneimittels zur Behandlung von Entzündungen.

2. Verwendung nach dem vorhergehenden Anspruch, **dadurch gekennzeichnet, daß** sich das Tripeptid am C-terminalen Ende des Peptids befindet.

3. Verwendung nach einem der vorhergehenden Ansprüche, **dadurch gekennzeichnet, daß** es sich bei dem Peptid um das Tripeptid Lysin-Prolin-Valin handelt, worin der Prolinrest in Form des optisch rechtsdrehenden Isomers (DPro) vorliegt.

4. Verwendung nach einem der vorhergehenden Ansprüche, **dadurch gekennzeichnet, daß** es sich bei dem Peptid um das Tripeptid Lysin-Prolin-Valin handelt, worin die Reste Lysin, Prolin und Valin in Form der optisch rechtsdrehenden Isomere (D-Lys-D-Pro-D-Val) vorliegen.

5. Verwendung nach einem der vorhergehenden Ansprüche, **dadurch gekennzeichnet, daß** das Peptid in geschützter Form oder in nicht geschützter Form vorliegt.

6. Verwendung nach dem vorhergehenden Anspruch, **dadurch gekennzeichnet, daß** der Schutz auf der Acylierung oder Acetylierung der Amino-Endgruppe oder auf der Amidbildung an der Carboxy-Endgruppe oder auf beiden Maßnahmen beruht.

7. Verwendung zur Herstellung eines Arzneimittels nach einem der Ansprüche 1 bis 6, **dadurch gekennzeichnet, daß** die Tripeptidsequenz Lysin-Prolin-Valin, worin der Prolinrest als optisch rechtsdrehendes Isomer (DPro) vorliegt, in einer Konzentration im Bereich von 10⁻¹² bis 1 M und vorzugsweise von 10⁻⁶ bis 10⁻¹ M verwendet wird.

## Claims

1. Use, for preparing a medicament, of an effective amount of at least one peptide containing the tripeptide lysine-proline-valine, in which the proline residue appears in the form of its dextrorotatory optical isomer (DPro), or of any functional biological equivalent, intended to treat inflammation.

2. Use according to the preceding claim, **characterized in that** the tripeptide is at the C-terminal end of the peptide.

3. Use according to one of the preceding claims, **characterized in that** the peptide is the tripeptide lysine-proline-valine in which the proline residue appears in the form of its dextrorotatory optical isomer (DPro).

4. Use according to one of the preceding claims, **characterized in that** the peptide is the tripeptide lysine-proline-valine in which the lysine, proline and valine residues appear in the form of dextrorotatory optical isomers (D-Lys-D-Pro-D-Val).

5. Use according to one of the preceding claims,
**characterized in that** the peptide is in a protected form or not.

6. Use according to the preceding claim, **characterized in that** the protection consists of a protection based either on acylation or acetylation of the amino-terminal end or on amidation of the carboxy-terminal end or on both.

7. Use, for preparing a medicament, according to any one of Claims 1 to 6, **characterized in that** the tripeptide lysine-proline-valine, in which the proline residue appears in the form of its dextrorotatory optical isomer (DPro), is used at a concentration of between 10⁻¹² M and 1 M, and preferably between 10⁻⁶ M and 10⁻¹ M.
